# EUROPÄISCHE PATENTANMELDUNG

(11) **EP 2 335 771 A1**
(43) Veröffentlichungstag der Anmeldung: **22.06.2011**
(21) Anmeldenummer: 09405230.5
(22) Anmeldetag: 21.12.2009
(51) Int. Cl.: A61M 37/00, A61D 7/00, A61B 17/34

(54) **Vorrichtung zur Injektion eines Feststoffes**

(71) Anmelder: forteq Nidau AG, 2560 Nidau (CH)
(72) Erfinder: Schmalz, Christian, 3250 Lyss (CH)
(74) Vertreter: Rüfenacht, Philipp Michael

(57) **Zusammenfassung**

Eine Vorrichtung (1) zum Injizieren eines Feststoffes (500) in einen menschlichen oder einen tierischen Körper (600) umfasst ein Gehäuse (100), einen Spritzenkörper (200) zur Aufnahme des Feststoffes (500), welcher mit einer Kanüle (220) verbunden ist, einen primären Stössel (230), welcher im Spritzenkörper (200) und in der Kanüle (220) verfahrbar ist, sowie einen sekundären Stössel (300), welcher im Gehäuse (100) verfahrbar ist. Die Vorrichtung (1) umfasst einen Kraftübertragungsmechanismus, welcher zwischen dem sekundären Stössel (300) und dem Spritzenkörper (200) und/oder der Kanüle (220) wirken kann, wobei durch eine Betätigung des sekundären Stössels (300) in distaler Richtung ein Verfahren des Spritzenkörpers (200) und/oder der Kanüle (220) in proximale Richtung bewirkbar ist.

## Beschreibung

Die Erfindung betrifft Vorrichtung zum Injizieren eines Feststoffes in einen menschlichen oder einen tierischen Körper, umfassend ein Gehäuse, einen Spritzenkörper zur Aufnahme des Feststoffes, wobei der Spritzenkörper mit einer Kanüle verbunden ist, einen primären Stössel, welcher im Spritzenkörper und in der Kanüle verfahrbar ist und einen sekundären Stössel, welcher im Gehäuse verfahrbar ist.

### Stand der Technik

Die Injektion von Feststoffen ist ein gängiges Verfahren bei der Langzeitmedikation (Depot-Injektion). Dazu wird ein Depot im Körper abgelegt, welches über eine vorbestimmte Zeitdauer, im Idealfall in konstanter und kontinuierlicher Weise, das Medikament an den Körper abgibt. Typische Anwendungen der Langzeitmedikation sind Schmerztherapien, Hormontherapien für die Empfängnisverhütung, etc. Weiter wird die Injektion von Feststoffen auch im Zusammenhang mit subkutanen Injektionen von Chips, zum Beispiel Mikroprozessoren oder Speicherchips, angewandt.

Eine Injektion eines Feststoffes erfolgt typischerweise, indem der Feststoff durch einen Stössel in einer im Körper befindlichen Kanüle gehalten wird, worauf die Kanüle bei ortsfestem Stössel zurückgezogen wird und so den Feststoff im Körper hinterlässt.

Die US 2009/0209903 A1 (S.C.R.A.S) zeigt eine solche Injektionsvorrichtung für intramuskuläre oder subkutane Injektionen von festen oder halbfesten Implantaten. Die Vorrichtung umfasst einen Hauptkörper, mit welchem eine Hohlnadel verbunden ist, und einen zweiten Körper, welcher koaxial im Hauptkörper angeordnet ist und die Hohlnadel umgibt, sowie einen Stössel, welcher koaxial in die Hohlnadel eingeführt werden kann. Der Stössel kann im zweiten Körper blockiert werden. Bei der Verwendung wird der Hauptkörper nach unten geführt, wodurch die Hohlnadel in die Haut eindringt, wobei gleichzeitig der Stössel mitgeführt wird. Mit dem Daumen wird der zweite Körper gegen die Haut gepresst, während mit dem Zeige- und Mittelfinger der Hauptkörper ergriffen und in proximaler Richtung zurückgezogen wird, wodurch der Hauptkörper entlang dem zweiten Körper mit der Hohlnadel, nicht aber mit dem Stössel nach oben fährt. Nachdem die Hohlnadel aus dem Körper geführt ist wird der Stössel im Hauptkörper eingerastet und aus dem Körper geführt.

Die bekannten Vorrichtungen zum Injizieren von Feststoffen haben den Nachteil, dass sie nicht intuitiv zu bedienen sind. Insbesondere müssen typischerweise mehrere unterschiedliche Aktionen durchgeführt werden, wie zum Beispiel Einfahren des Stössels, Zurückziehen der Kanüle ohne den Stössel, Zurückziehen des Stössels etc. Für den ungeübten Verwender solcher Vorrichtungen stellt dies eine beachtliche Herausforderung dar. Weiter können bei unsachgemässer Handhabung einer solchen Vorrichtung dem zu Injizierenden zusätzliche zu den beim Einführen der Kanüle verursachten Schmerzen zugefügt werden.

Bis anhin musste der Verwender den Stössel nach dem Einführen der Kanüle in den Körper und nachdem der Feststoff in der Kanüle platziert wurde, ortsfest halten und gleichzeitig die Kanüle zurückziehen. Diese Bewegung ist insofern problematisch, respektive schwierig durchzuführen, als dass die Vorrichtung bezüglich einer proximalen Bewegung keinen Anschlag aufweist, welcher verhindert, dass der primäre Stössel gleichzeitig mit dem Spritzenkörper und/oder der Kanüle zurückgezogen wird, wodurch der Feststoff unter Umständen nicht an seinem vorgesehenen Platz verbleibt. Dies wurde einzig dadurch verhindert, dass der Verwender den Stössel relativ zum Körper ortsfest gehalten hat. Ein Mensch oder ein Tier verhält sich aber während der Injektion typischerweise nicht ruhig. Alleine der ungewohnte Vorgang des Einführens eines Feststoffes in das Gewebe führt bei Menschen oft zu Anspannungen und unter Umständen zu unkontrollierten Bewegungen, wie Zucken oder Zittern. Zusätzlich erfährt der Mensch oder das Tier beim Einführen der Kanüle unter die Haut auch Schmerz, insbesondere wenn auf eine lokale Betäubung der Einstichstelle verzichtet wird, was ein weiterer Grund für unkontrollierte Bewegungen ist. Diese unkontrollierten Bewegungen führen für den Verwender zu Problemen bei der Injektion, da dieser die Bewegungen des Körpers parallel und synchron mit dem Stössel nachführen und die Kanüle gleichzeitig zurückziehen muss.

### Darstellung der Erfindung

Aufgabe der Erfindung ist es, eine dem eingangs genannten technischen Gebiet zugehörende Vorrichtung zum Injizieren eines Feststoffes zu schaffen, welche einfach, intuitiv und sicher zu bedienen und zudem kostengünstig und einfach aufgebaut ist.

Die Lösung der Aufgabe ist durch die Merkmale des Anspruchs 1 definiert. Gemäss der Erfindung umfasst die Vorrichtung einen Kraftübertragungsmechanismus, welcher zwischen dem sekundären Stössel und dem Spritzenkörper und/oder der Kanüle wirken kann, wobei durch eine Betätigung des sekundären Stössels in distaler Richtung ein Verfahren des Spritzenkörpers und/oder der Kanüle in proximaler Richtung bewirkbar ist.

Der Begriff "distal" bezeichnet dabei im Folgenden eine Richtung zum Körper hin und der Begriff "proximal" bezeichnet die dazu entgegengesetzte Richtung, respektive vom Körper weg. In Relation zur Vorrichtung sind die beiden Richtungen als jeweils parallel zu einer Längsachse zu verstehen, wobei die Längsachse zum Beispiel durch das Gehäuse, den Spritzenkörper, die Kanüle oder durch einen der beiden Stössel definiert werden kann. Falls nicht anders gekennzeichnet sind die Richtungen "distal" und "proximal" als parallel zu einer Längsachse des primären Stössels zu verstehen. Die Kanüle ragt bei der Anwendung der Vorrichtung zumindest zeitweise in distaler Richtung aus dem Gehäuse heraus.

Nachdem die Kanüle in den Körper geführt und der Feststoff mit dem primären Stössel in der Kanüle platziert ist, wird typischerweise der Spritzenkörper und/oder die Kanüle bei bezüglich des Körpers ortsfestem primärem Stössel zurückgezogen, so dass der Feststoff im Körper verbleibt. Durch den Kraftübertragungsmechanismus zwischen dem sekundären Stössel und dem Spritzenkörper wird nun eine Vorrichtung geschaffen, mit welcher durch ein Verschieben des sekundären Stössels in distale Richtung der Spritzenkörper und/oder die Kanüle in proximaler Richtung verfahren werden kann. Dadurch wird eine besonders ergonomische Bedienung der Vorrichtung erreicht, da bei im Körper befindlicher Kanüle, insbesondere bei vollständig, bis zum Anschlag am Gehäuse im Körper befindlicher Kanüle unkontrollierte Bewegungen des Körpers mühelos durch einen leichten Druck in distaler Richtung, insbesondere durch ein Einführen des sekundären Stössels, aufgenommen werden können und so die gewünschte Position des Feststoffes beibehalten werden kann. Des Weiteren wird damit erreicht, dass das Zurückziehen der Kanüle durch die ergonomisch und intuitiv bevorzugte distale Bewegung des sekundären Stössels erreicht werden kann.

Bevorzugt umfasst das Gehäuse insbesondere in einem proximalen Bereich Halteelemente. Damit wird erreicht, dass die Vorrichtung wie eine herkömmliche Spritze gehalten werden kann, nämlich indem mittels Zeige- und Mittelfinger die Halteelemente und mittels des Daumens der Stössel, insbesondere der sekundäre Stössel, geführt werden. Die Halteelemente können als zwei gegenüberliegende, radial nach aussen ragende Elemente oder als ein umlaufender Ring ausgebildet sein. Dem Fachmann sind auch weitere Möglichkeiten bekannt, wie solche Halteelemente in ergonomischer Weise realisiert werden können.

Vorzugsweise umfasst die Vorrichtung ein Zahnstangengetriebe. Das Zahnstangengetriebe kann so in der Vorrichtung eingebaut sein, dass eine distale Bewegung des sekundären Stössels in eine proximale Bewegung des Spritzenkörpers und/oder der Kanüle umgewandelt werden kann, womit eine mögliche Realisierung des Kraftübertragungsmechanismus erreicht wird. Dies hat den Vorteil, dass die Vorrichtung wie eine herkömmliche Spritze bedient werden kann, ohne dass für das Zurückziehen des Spritzenkörpers und/oder der Kanüle ein Element der Vorrichtung in die dem Anwender ungewohnte distale Richtung geführt werden muss. Damit kann die Relativbewegung zwischen dem Spritzenkörper (und/oder der Kanüle) und dem sekundären Stössel in besonders einfacher Weise realisiert werden, insbesondere da Zahnstangen und Zahnräder kostengünstig und einfach herzustellen sowie einfach zu montieren sind. Des Weiteren stellt ein Zahnstangengetriebe sicher, dass die Bewegungen des sekundären Stössels und des Spritzenkörpers und/oder der Kanüle klar definiert sind, womit die Bedienung der Vorrichtung weiter vereinfacht und die Störungsanfälligkeit weiter reduziert wird.

Alternativ kann auch auf ein Zahnstangengetriebe verzichtet werden, wobei die Kraftübertragung anderweitig realisiert wird (siehe unten).

Bevorzugt ist der Kraftübertragungsmechanismus als ein Zahnstangengetriebe ausgebildet, welches eine erste Zahnstange, eine zweite Zahnstange und ein Zahnrad umfasst, wobei
a) das Zahnrad frei rotierbar im Gehäuse gelagert ist; und
b) die erste Zahnstange mit dem Spritzenkörper verbunden ist; und
c) die zweite Zahnstange mit dem sekundären Stössel verbunden ist; wobei
d) das Zahnrad mit der ersten und der zweiten Zahnstange zusammenwirkt.

Dazu können die erste Zahnstange einstückig mit dem Spritzenkörper und/oder die zweite Zahnstange einstückig mit dem sekundären Stössel ausgebildet sein. Damit wird ein kostengünstiger und einfacher Aufbau der Vorrichtung erreicht. Das Zahnrad wird bevorzugt separat, als einzelnes Element hergestellt. Die Lagerung des Zahnrades erfolgt dabei zum Beispiel auf einem Stift, welcher fest mit dem Gehäuse verbunden ist, wobei für die Befestigung des Zahnrades am Stift zum Beispiel eine dem Fachmann bekannte Clipverbindung vorgesehen sein kann. Bevorzugt ist auch der Stift einstückig mit dem Gehäuse verbunden, um weiter einen einfachen Aufbau der Vorrichtung zu erreichen.

Alternativ kann der Kraftübertragungsmechanismus auch anders ausgebildet sein. Zum Beispiel könnte statt des Zahnrades und der Zahnstangen auch ein zahnloses Rad vorgesehen sein, welches über Reibung mit entsprechenden Bereichen des sekundären Stössels und des Spritzenkörpers zusammenwirken kann, wobei aber ein gewisser Anpressdruck zwischen dem zahnlosen Rad und den entsprechenden Bereichen gewährleistet sein muss. Weiter kann als Kraftübertragungsmechanismus auch eine Verbindung des Spritzenkörpers und/oder der Kanüle über eine Umlenkung mit dem sekundären Stössel erfolgen. Die Umlenkung kann dazu in einem proximalen Bereich des Gehäuses vorgesehen und mit diesem verbunden sein. Für die Umlenkung kann ein rechtwinklig zur Längsrichtung der Vorrichtung ein Stift oder eine Rolle vorgesehen sein. Zur Übertragung der Bewegung kann ein Faden, ein Draht, ein Band oder ähnliches verwendet werden, welche(s) an einem ersten Ende mit dem Spritzenkörper oder der Kanüle und mit einem zweiten Ende mit dem sekundären Stössel verbunden ist und proximal um den Stift oder die Rolle umgelenkt wird. Schliesslich kann die Rückführung der Kanüle auch mit einer Feder erfolgen, wobei die Kanüle bei gespannter Feder aus dem Gehäuse hinausragt und bei entspannter Feder nur teilweise, insbesondere nicht, aus dem Gehäuse hinausragt. Diese Feder würde beim Einfahren des sekundären Stössels gespannt und zur Rückführung der Kanüle entspannt und so eine weitere Möglichkeit eines Kraftübertragungsmechanismus darstellen. Bei einer solchen Rückführung ist gegebenenfalls zu verhindern, dass die Kanüle nach Überwindung einer Haftreibung im Körper zu schnell aus demselben zurückgezogen wird. Es könnte dazu eine Bremse vorgesehen sein, womit die Rückführgeschwindigkeit kontrolliert werden könnte. Zum Beispiel wäre ein seitlich am Gehäuse angeordnetes federndes Element, welches bei Druckbeaufschlagung mit einem Finger den Spritzenkörper festklemmen kann, eine denkbare Umsetzung einer solchen Bremse. Dem Fachmann sind dazu auch weitere Möglichkeiten bekannt, mit welchen eine einfache Umsetzung der proximalen Rückführung der Kanüle umsetzbar ist.

Die Vorrichtung umfasst vorzugsweise eine erste Verriegelungsvorrichtung, womit der sekundäre Stössel bezüglich einer axialen Verfahrbarkeit im Gehäuse blockierbar ist. Bevorzugt wird zuerst die Kanüle in den Körper eingeführt und anschliessend über den primären Stössels der Feststoff in die Kanüle geführt. Erst nachdem der Feststoff den vorgesehenen Platz in der Kanüle, insbesondere in einem Bereich der Kanüle, welcher aus dem Gehäuse hinausragt, eingenommen hat, wird die erste Verriegelungsvorrichtung entriegelt, womit ein Einfahren des sekundären Stössels ermöglicht wird. Damit wird erreicht, dass der sekundäre Stössel nicht unbeabsichtigt verfahren werden kann, insbesondere wird damit verhindert, dass der Spritzenkörper und/oder die Kanüle zurückgefahren werden, bevor der Feststoff in der Kanüle platziert ist. Falls nämlich der primäre Stössel zu wenig weit vorgefahren ist und trotzdem der sekundäre Stössel betätigt werden könnte, so bestünde die Gefahr, dass gleichzeitig der primäre Stössel und der Feststoff distal und die Kanüle proximal verfahren würden, womit der typischerweise stumpfe Feststoff in den Körper eingepresst wird, was dem zu Injizierenden erhebliche Schmerzen verursacht. Die erste Verriegelungsvorrichtung umfasst bevorzugt ein federndes Element, welches mit dem Gehäuse verbunden ist, und eine Ausnehmung im sekundären Stössel, wobei das federnde Element mit der Ausnehmung in Eingriff stehen kann. Vorzugsweise steht das federnde Element in entspanntem Zustand in Eingriff mit der Ausnehmung und muss mit einer Kraft beaufschlagt werden, wenn es aus der Ausnehmung bewegt werden soll.

Alternativ kann die erste Verriegelungsvorrichtung auch anders ausgebildet sein oder es kann auf die erste Verriegelungsvorrichtung ganz verzichtet werden. In letzterem Fall muss der Verwender darauf bedacht sein, dass der sekundäre Stössel erst dann distal in das Gehäuse eingefahren werden soll, nachdem der primäre Stössel vollständig distal eingefahren ist.

Vorzugsweise ist die erste Verriegelungsvorrichtung durch den primären Stössel, insbesondere durch eine distale Verschiebung des primären Stössels, deaktivierbar. Bevorzugt erfolgt die Deaktivierung der Verriegelungsvorrichtung nach dem vollständigen distalen Verfahren des primären Stössels, d.h. nachdem der Feststoff in der Kanüle platziert ist. Damit wird eine besonders einfache Bedienung der Vorrichtung erreicht. Bevorzugt ist nämlich der primäre Stössel distal vollständig durch den Spritzenkörper hindurch in die Kanüle verfahren, bevor der sekundäre Stössel betätigt wird. Die Kanüle soll ja erst dann zurückgezogen werden, nachdem der Feststoff in derselben platziert worden ist. Mit der Deaktivierung der ersten Verriegelungsvorrichtung wird also gewissermassen ein bevorzugter Ablauf sequentiell automatisch koordiniert, so dass nicht versehentlich vor dem Einführen des Feststoffes der Spritzenkörper und/oder die Kanüle zurückgefahren werden können. Der Stösselkopf des primären Stössels ist dazu bevorzugt so ausgebildet, dass damit das federnde Element aus der Ausnehmung des sekundären Stössels gehoben werden kann.

Alternativ kann die Deaktivierung der ersten Verriegelungsvorrichtung auch anderweitig erfolgen. Die Deaktivierung könnte zum Beispiel direkt von Hand erfolgen, nachdem der primäre Stössel den Feststoff in die Kanüle überführt hat. Die Deaktivierung kann zum Beispiel durch einen radial einführbaren Riegel oder auf andere dem Fachmann bekannte Weisen erfolgen.

Bevorzugt umfasst die Vorrichtung einen ersten Energiespeicher, wobei Energie des ersten Energiespeichers zur distalen Verschiebung des primären Stössels einsetzbar ist. Damit wird erreicht, dass der Verwender in besonders einfacher Weise den Feststoff in der Kanüle platzieren kann, ohne dass dieser, abgesehen von einer Aktivierung des Energiespeichers, selbst eine Energie aufwenden muss. Weiter kann der Verwender nicht kontrollieren, wo sich der Feststoff in der Kanüle befindet, woraus sich gewisse Unsicherheiten bei der Verwendung ergeben können. Durch die Ausbildung eines ersten Energiespeichers für die Betätigung des primären Stössels wird eine automatische Einführung des Feststoffes in die Kanüle erreicht, womit dem Verwender die obig genannten Unsicherheiten genommen werden können. Typischerweise erfolgt die Platzierung des Feststoffes in der Kanüle nämlich, nachdem die Kanüle in den Körper eingeführt worden ist. Die Verwendung eines ersten Energiespeichers ist auch deshalb von Vorteil, weil die Platzierung des Feststoffes in der Kanüle damit relativ schnell erfolgen kann, womit die Dauer der Injektion, insbesondere die Zeitspanne, in welcher die Kanüle im Körper steckt, verringert werden kann. Dies kommt offensichtlich dem zu Injizierenden entgegen. Natürlich kann die Vorrichtung auch für ein distales Verschieben des primären Stössels vor dem Einführen der Kanüle in den Körper vorgesehen sein. Um in diesem Fall zu verhindern, dass der Feststoff aus der Kanüle fällt, kann entweder die Leistung der Energieabgabe des Energiespeichers entsprechend gewählt werden, so dass der Feststoff nicht zu stark beschleunigt wird und/oder eine Innenwandung der Kanüle und der Feststoff sind so aufeinander abzustimmen, dass genügend Reibung vorherrscht, so dass der Feststoff durch die Reibung an einem Austritt aus der Kanüle gehindert werden kann.

Alternativ kann auch auf den ersten Energiespeicher verzichtet werden. In diesem Fall wird der primäre Stössel von Hand betätigt.

Der erste Energiespeicher umfasst bevorzugt eine erste Feder, insbesondere zur Verschiebung des primären Stössels in distaler Richtung, wobei die erste Feder insbesondere als Schraubenfeder ausgebildet ist. Der Einsatz einer Feder als erster Energiespeicher hat den Vorteil, dass die notwendige Leistung durch die Wahl des Federtyps optimiert werden kann. Weiter ergibt sich daraus auch die Möglichkeit, eine Feder zu wählen, welche ein nicht-lineares Kraft/Weg-Verhalten aufweist. Dies kann dann von Vorteil sein, wenn zu Beginn des Verschiebens des Feststoffes eine Haftreibung überwunden werden muss. Bevorzugt wird eine Schraubenfeder, insbesondere eine Schraubendruckfeder eingesetzt, da diese in besonders hoher Vielfalt auf dem Markt zur Verfügung stehen und damit auch kostengünstig erhältlich sind.

Alternativ können auch andere Ausbildungen des Energiespeichers vorgesehen sein. Zum Beispiel könnten elastische Zugmittel zwischen dem primären Stössel und dem Gehäuse vorgesehen sein, welche beispielsweise als Zugfeder, Gummiband oder Ähnliches ausgebildet sein können. Auch zur Schraubenfeder stehen eine Vielzahl dem Fachmann bekannte Alternativen zur Verfügung. Je nach Geometrie der Vorrichtung kann es zum Beispiel von Vorteil sein, eine Kegelfeder vorzusehen.

Der primäre Stössel ist bevorzugt im sekundären Stössel geführt. Damit wird wiederum ein einfacher Aufbau der Vorrichtung erreicht. Dazu ist der sekundäre Stössel bevorzugt als Hohlkörper ausgebildet, insbesondere als im Wesentlichen einseitig geschlossener Hohlzylinder. Der primäre Stössel weist dabei einen länglichen stabförmigen Abschnitt, welcher durch den Spritzenkörper und die Kanüle führbar ist, und am proximalen Ende einen Kontaktbereich auf, welcher zum Beispiel scheibenförmig ausgebildet und rechtwinklig zu einer Längsachse des sekundären Stössels orientiert sein kann Der sekundäre Stössel kann inwändig axial eine Nut aufweisen, welche mit einem entsprechenden vorstehenden Element des Kontaktbereichs des primären Stössels zusammenwirkt, um eine Verdrehung desselben relativ zum primären Stössel zu verhindern.

Alternativ können der primäre und der sekundäre Stössel auch nebeneinander im Gehäuse geführt sein. Der Kontaktbereich muss nicht scheibenförmig ausgebildet sein, sondern muss lediglich geeignet ausgebildet sein, um mittels der ersten Feder den primären Stössel mit einer Kraft beaufschlagen zu können (siehe unten). Dazu kann es beispielsweise ausreichen, dass der primäre Stössel an einem proximalen Ende ein Loch aufweist, in welches das distale Ende der ersten Feder hindurchgeführt werden kann, womit die Kraftübertragung der ersten Feder auch gewährleistet ist. In diesem Fall ist der primäre Stössel bezüglich des sekundären Stössels bevorzugt verdrehgesichert.

Vorzugsweise ist die erste Feder im sekundären Stössel so angeordnet, dass der primäre Stössel durch eine Federkraft der ersten Feder aus dem sekundären Stössel axial verfahrbar ist. Die erste Feder wird in den Hohlraum des sekundären Stössels geführt und der primäre Stössel wird anschliessend in den Hohlraum eingeführt, so dass die Feder gewissermassen zwischen dem sekundären Stössel und dem Kontaktbereich des primären Stössels eingeklemmt und spannbar ist. Damit kann der gesamte für das Verfahren des primären Stössels benötigte Federweg im sekundären Stössel untergebracht werden, womit eine platzsparende Konstruktion der Vorrichtung erreicht wird.

Alternativ könnte der primäre Stössel auch zusammen mit dem sekundären Stössel eingefahren werden, womit auf die erste Feder verzichtet werden kann. Dazu ist vorzugsweise der Kraftübertragungsmechanismus deaktivierbar ausgebildet. Dies kann beispielsweise durch ein axiales Verdrehen des sekundären Stössels erreicht werden, wobei die zweite Zahnstange nicht im Eingriff mit dem Zahnrad steht. Der sekundäre Stössel wird dabei distal verfahren, womit gleichzeitig der primäre Stössel durch den Spritzenkörper hindurch in die Kanüle verfahren wird. Anschliessend wird der sekundäre Stössel bei ortsfestem primärem Stössel zurückgefahren und axial so gedreht, so dass dessen zweite Zahnstange wieder in Eingriff mit dem Zahnrad steht. Dazu könnte weiter eine Zwangsführung mittels Nuten und vorstehendem Element vorgesehen sein. Schliesslich könnte der primäre Stössel auch länger ausgebildet sein, so dass ein Stösselkopf des primären Stössels um mindestens eine Distanz, welche der Distanz entspricht, um welche der Feststoff verfahren werden muss, aus dem sekundären Stössel proximal herausragt. Damit könnte zuerst der primäre Stössel distal von Hand in das Gehäuse geschoben und damit der Feststoff in der Kanüle platziert werden und anschliessend der sekundäre Stössel bei ortsfestem primären Stössel betätigt werden, um die Kanüle zurück zu fahren.

Bevorzugt umfasst die Vorrichtung eine zweite Verriegelungsvorrichtung, mit welcher der primäre Stössel bezüglich des sekundären Stössels, insbesondere bei gespannter erster Feder, verriegelbar ist. Durch ein Deaktivieren der zweiten Verriegelungsvorrichtung entspannt sich die zweite Feder und führt den primären Stössel aus dem sekundären Stössel heraus. Die Verriegelungsvorrichtung umfasst bevorzugt ein radial betätigbarers federndes Element, welches mit dem sekundären Stössel, insbesondere einstückig, ausgebildet ist. Das federnde Element umfasst ein, axial nach innen ragendes, erstes vorstehendes Element, welches bei aktiver Verriegelungsvorrichtung in Eingriff mit einem Kontaktbereich des primären Stössels steht. Bei gespannter erster Feder liegt der Kontaktbereich des primären Stössels proximal zum ersten vorstehenden Element und wirkt so mit diesem zusammen. Bei entspannter Feder befindet sich das vorstehende Element proximal, hinter dem Kontaktbereich des primären Stössels.

Dem Fachmann ist eine Vielzahl von weiteren geeigneten Verriegelungsvorrichtungen bekannt, welche für den vorliegenden Fall auch geeignet sind. Zum Beispiel könnte das erste vorstehende Element statt mit dem Kontaktbereich des Stössels auch mit der Feder zusammenwirken. Falls der primäre Stössel von Hand betätigt wird, kann auf die zweite Verriegelungsvorrichtung auch verzichtet werden.

Vorzugsweise umfasst der sekundäre Stössel ein Betätigungselement, mit welchem die zweite Verriegelungsvorrichtung von Hand entriegelbar ist. Das Betätigungselement ist dabei so ausgebildet, dass es mit dem federnden Element zusammenwirken, insbesondere das erste vorstehende Element radial nach aussen treiben kann.

Das Betätigungselement ist bevorzugt an einem proximalen Ende des sekundären Stössels angeordnet und insbesondere als Druckknopf ausgebildet. Dazu weist die zweite Verriegelungsvorrichtung bevorzugt ein federndes Element mit einem zweiten vorstehenden Element auf, welches einen bezüglich einer axialen Richtung abgeschrägten Bereich aufweist. Das zweite vorstehende Element ist bevorzugt bei gespannter Feder proximal zum Kontaktbereich des primären Stössels angeordnet. Eine Entriegelung der zweiten Verriegelungsvorrichtung kann durch einen Stift erfolgen, welcher axial in Richtung des abgeschrägten Bereichs verfahrbar ist und insbesondere mit dem Betätigungselement verbunden ist. Beim distalen Verfahren des Stiftes kontaktiert dieser den abgeschrägten Bereich des zweiten vorstehenden Elements des federnden Elements und treibt letzteres radial nach aussen. Anschliessend entspannt sich die erste Feder und führt den primären Stössel durch den Spritzenkörper hindurch in die Kanüle. Der Stift ist bevorzugt mit einem einseitig geschlossenen Zylinder verbunden, welcher passförmig über das proximale Ende des sekundären Stössels geführt werden kann und ist bevorzugt rechtwinklig im Zylinder mit dem Boden verbunden, wobei der sekundäre Stössel eine entsprechende Öffnung aufweist, durch welche der Stift hindurchführbar ist.

Alternativ könnte die zweite Verriegelungsvorrichtung auch mittels Fernwirkung durch das Einführen der Kanüle in den Körper aktiviert werden. Dazu könnte am distalen Ende des Gehäuses, insbesondere am Gehäuseboden, ein axial verschiebbares Element vorgesehen sein, welches mit der zweiten Verriegelungsvorrichtung zusammenwirken kann. Weiter kann das Betätigungselement auch seitlich am sekundären Stössel vorgesehen sein. Dazu kann das federnde Element zum Beispiel stabförmig ausgebildet und mittig mit dem sekundären Stössel verbunden sein, so dass mittels einer Druckbeaufschlagung eine Kippbewegung erreichbar ist, womit das erste vorstehende Element radial nach aussen geführt werden kann. Dem Fachmann sind natürlich auch weitere geeignete Ausbildungen eines Betätigungselements bekannt.

Bevorzugt umfasst die Vorrichtung eine Hülse, welche axial verfahrbar im distalen Ende des Gehäuses gelagert ist und in einem ersten Zustand im Wesentlichen innerhalb des Gehäuses angeordnet ist und in einem zweiten Zustand zumindest teilweise aus dem Gehäuse hinausragt. Das distale Ende des primären Stössels ist typischerweise nach der Injektion kontaminiert, d.h. es können Gewebereste, Körperflüssigkeiten, wie z.B. Blut, am primären Stössel haften. Durch die Ausbildung einer verfahrbaren Hülse am distalen Ende des Gehäuses kann nun das aus dem Gehäuse ragende Ende des primären Stössels abgedeckt werden, so dass der Verwender, der zu Injizierende und Drittpersonen mit dem kontaminierten Bereich nicht in Kontakt kommen und insbesondere nicht mit Krankheitserregern oder ähnlichem infiziert werden können. Weiter kann damit auch verhindert werden, dass Gegenstände oder Ablageflächen kontaminiert werden. Die Hülse liegt dabei nach der Injektion bevorzugt im zweiten Zustand vor. Sie kann aber auch bis unmittelbar vor der Injektion im zweiten Zustand sein, so dass die Kanüle geschützt ist und damit auch ungewollten Verletzungen vorgebeugt wird. Nach der vollzogenen Injektion des Feststoffes ragt lediglich der primäre Stössel über das distale Ende des Gehäuses hinaus.

Alternativ kann die Hülse auch aussen am Gehäuse axial verfahrbar gelagert sein.

Die Hülse ist bevorzugt als im Wesentlichen einseitig geschlossener Zylinder, insbesondere als Hohlzylinder ausgebildet. Am Boden des Zylinders befindet sich eine Öffnung, welche genügend gross ist, so dass die Kanüle durchgeführt werden kann. Die Hülse weist weiter seitliche, axial orientierte Einschnitte auf, über welche die Hülse im Gehäuse axial verfahrbar geführt ist.

Statt der Hülse kann auch eine Schutzkappe vorgesehen sein, welche über das distale Ende des Gehäuses überstülpbar ist und so die Kanüle, respektive den Verwender und Drittpersonen schützen kann. Die Schutzkappe wird vor der Verwendung der Vorrichtung abgenommen und kann nach der Verwendung wieder übergestülpt werden. Alternativ kann auf die verfahrbare Hülse auch verzichtet werden.

Bevorzugt umfasst die Vorrichtung einen zweiten Energiespeicher, wobei Energie des zweiten Energiespeichers zur distalen Verschiebung der Hülse einsetzbar ist. Bevorzugt wird der zweite Energiespeicher vor dem Hinausziehen des primären Stössels aus dem Körper aktiviert, so dass die Hülse gegen den Körper gepresst wird, währenddem der primäre Stössel aus dem Körper gezogen wird. Damit wird eine Kontamination schon während des Hinausziehens des primären Stössels verhindert.

Alternativ kann die Hülse auch von Hand betätigbar ausgebildet sein. Dazu kann die Hülse auch ausserhalb des Gehäuses axial verschiebbar gelagert sein, so dass die Hülse ergriffen und verschoben werden kann. Weiter können auch Rastungen vorgesehen sein, womit die Hülse im ersten und zweiten Zustand eingerastet werden kann.

Vorzugsweise liegt in einem Zustand, insbesondere nach der Verwendung der Vorrichtung, die Kanüle vollständig innerhalb des Gehäuses, wobei der primäre Stössel distal und die Hülse teilweise aus dem Gehäuse hinaus ragen und wobei die Hülse einen aus dem Gehäuse hinausragenden Bereich des primären Stössels vollständig abdeckt. Damit wird sowohl der Verwender wie auch der Injizierte vor einer Verletzung durch die Kanülenspitze wie auch vor einer Kontamination geschützt.

Bevorzugt umfasst ein zweiter Energiespeicher eine zweite Feder, welche zwischen dem Gehäuse und der Hülse wirkbar ist und insbesondere im ersten Zustand gespannt ist. Die zweite Feder ist bevorzugt gespannt, bis der Spritzenkörper und/oder die Kanüle vollständig zurückgezogen sind. Während sich die zweite Feder entspannt, befindet sich das distale Ende des primären Stössels typischerweise noch immer im Körper. Die zweite Feder presst dann die Hülse vorerst an den Körper. Erst, wenn die Vorrichtung, insbesondere der primäre Stössel, aus dem Körper entfernt wird, wird zwischen dem Gehäuse und dem Körper genügend Platz geschaffen, womit die Hülse mittels der zweiten Feder aus dem Gehäuse geführt werden kann. Während des Entfernens des primären Stössels aus dem Körper sind dadurch kontaminierte Bereiche der Vorrichtung immer abgeschirmt, womit eine sichere Injektion eines Feststoffes erreicht werden kann. Insbesondere im Vergleich mit einer von Hand betätigbaren Hülse muss der Verwender dadurch nicht im Bereich der Kanüle oder des kontaminierten Endes des primären Stössels hantieren.

Alternativ kann auch ein anderer zweiter Energiespeicher vorgesehen sein. Dieser kann zum Beispiel mittels eines spannbaren elastischen Elements, wie beispielsweise einem Gummiband realisiert sein. Schliesslich kann auf den zweiten Energiespeicher auch verzichtet werden, wobei die Hülse von Hand betätigt wird. Dabei müsste der Anwender aber darauf bedacht sein, dass er sich im Bereich des distalen Endes der Vorrichtung, nach deren Verwendung, kontaminieren könnte.

Vorzugsweise ist die zweite Feder als Schraubenfeder ausgebildet. Die Schraubenfeder hat den Vorteil, dass diese kostengünstig und konstruktiv einfach in die Vorrichtung einbaubar ist.

Alternativ können auch andere dem Fachmann bekannte Federn, wie beispielsweise Kegelfedern eingesetzt werden, insbesondere kann die Federform auch dem Gehäuse, respektive der Hülse angepasst sein.

Die Vorrichtung umfasst bevorzugt eine dritte Verriegelungsvorrichtung, womit die Hülse bezüglich einer axialen Verfahrbarkeit im Gehäuse verriegelbar ist. Dazu umfasst die Hülse an einem proximalen Aussenbereich radial abstehende federnde Elemente, welche in entsprechenden Ausnehmungen des Gehäuses einrasten können. Um das Federn der Elemente zu realisieren, kann die Hülse aus einem zumindest geringfügig federnden Material ausgebildet sein, womit die federnden Elemente einstückig mit der Hülse ausgebildet werden können. Damit wird eine kostengünstige Herstellung ermöglicht.

Alternativ kann die Hülse auch im Grundzustand mittels der Feder soweit vorgefahren sein, dass die Kanüle vollständig durch die Hülse abgedeckt ist und nur durch Beaufschlagung einer Kraft proximal zurückgefahren wird, insbesondere beim Anpressen an den Körper, womit auf die dritte Verriegelungsvorrichtung verzichtet werden kann. Allerdings gestaltet sich dadurch das Einführen der Kanüle, insbesondere ein bezüglich der Körperoberfläche nicht senkrechtes Einführen der Kanüle, schwieriger. Durch die Hülse kann der Einstich-Ort nicht genau lokalisiert werden. Weiter wird bei einer nicht senkrechten Einführung der Kanüle die Hülse mit einer radialen Kraft beaufschlagt, wodurch sich diese verkanten kann.

Bevorzugt ist die dritte Verriegelungsvorrichtung im ersten Zustand entriegelbar und insbesondere im zweiten Zustand nicht entriegelbar. Im ersten Zustand ist die dritte Verrieglungsvorrichtung vorzugsweise entriegelbar, so dass die Hülse über den zweiten Energiespeicher, insbesondere durch die zweite Feder distal verfahren werden kann, so dass der primäre Stössel und/oder die Kanüle abgedeckt wird. Falls die Hülse erst nach vollendeter Injektion nach vorne geführt wird, ist es von Vorteil, wenn die Hülse im vorgefahrenen Zustand mittels der dritten Verriegelungsvorrichtung irreversibel verriegelt ist, insbesondere nicht durch einfache Mittel entriegelt werden kann. Damit wird das Risiko einer Kontamination verringert, da so durch unsachgemässe Handhabung die Hülse nicht versehentlich zurückgeführt werden und den kontaminierten Bereich offen legen kann. Je nach Ausbildung der Hülse kann damit auch verhindert werden, dass die Vorrichtung wieder verwendet wird. Dazu kann die Hülse genügend lang ausgebildet sein, so dass, wenn der sekundäre Stössel zurückgezogen würde, die Kanüle nicht aus der Hülse hinausragen kann. Es wäre auch denkbar, die Öffnung in der Hülse ähnlich einem Rückschlagventil mit einer federnden Klappe zu versehen, welche während der Verwendung durch die Kanüle oder den Stössel entgegen der Federkraft offen gehalten wird und sich, nachdem die Hülse über den primären Stössel hinausgefahren ist, schliesst.

Alternativ, insbesondere wenn die Vorrichtung für den mehrmaligen Gebrauch vorgesehen ist, kann die dritte Verriegelungsvorrichtung im zweiten Zustand auch entriegelbar ausgebildet sein. Falls sich die Hülse standardmässig im zweiten Zustand befindet, kann die dritte Verriegelungsvorrichtung auch ausschliesslich im zweiten Zustand ver- und entriegelbar sein. Schliesslich kann auch ganz auf die dritte Verriegelungsvorrichtung verzichtet werden.

Die dritte Verriegelungsvorrichtung ist vorzugsweise durch den sekundären Stössel deaktivierbar ausgebildet. Dies hat den Vorteil, dass der Verwender diese nicht aktiv, respektive bewusst deaktivieren muss und damit auch nicht vergessen kann, diese zu deaktivieren. Bevorzugt erfolgt die Deaktivierung der dritten Verriegelungsvorrichtung beim vollständigen Einführen des sekundären Stössels, insbesondere da in diesem Zustand die Kanüle vollständig zurückgeführt und die Injektion gewissermassen abgeschlossen ist. Falls durch den zweiten Energiespeicher keine grosse Kraft auf die Hülse wirkt, kann die dritte Verriegelungsvorrichtung auch schon vorher deaktiviert werden. Bevorzugt erfolgt aber die Deaktivierung der dritten Verriegelungsvorrichtung frühestens nach dem Einführen der Kanüle in den Körper.

Die Hülse umfasst an einem proximalen Bereich mindestens ein nach aussen gerichtetes Element, welches aber auch um die Hülse umlaufend vorgesehen sein kann. Ein distaler Bereich des Elements weist eine Flanke auf, welche senkrecht auf der Hülse steht und damit mit entsprechenden Ausnehmungen oder radial nach innen gerichteten Elementen des Gehäuses zusammenwirken kann. Ein proximaler Bereich des Elements weist eine abgeschrägte Flanke auf, welche in distaler Richtung radial nach aussen strebt. Der sekundäre Stössel ist so ausgebildet, dass er mit der abgeschrägten Flanke zusammenwirken und das nach aussen gerichtete Element der Hülse radial nach innen, insbesondere aus der Nut, respektive aus dem Eingriff mit dem radial nach innen gerichteten Element des Gehäuses treiben kann. Über die zweite Feder kann anschliessend die Hülse in distaler Richtung verfahren werden.

Falls die Deaktivierung unmittelbar nach dem Einführen der Kanüle in den Körper erfolgen soll, kann ein Betätigungselement zum Beispiel in einem distalen Bereich des Gehäuses der Vorrichtung, insbesondere gerade in einem Kontaktbereich des Gehäuses mit dem Körper, oder an der Hülse selbst vorgesehen sein.

Bevorzugt umfasst der Spritzenkörper eine Rückhaltevorrichtung zum Zurückhalten des Feststoffes vor der Injektion. Während des Transports oder vor dem Einführen der Kanüle in den Körper ist die Vorrichtung typischerweise Erschütterungen ausgesetzt, wodurch der Feststoff aus dem Spritzenkörper herausrutschen kann. Durch die Ausbildung einer Rückhaltevorrichtung wird nun erreicht, dass der Feststoff vor der Verwendung der Vorrichtung, insbesondere vor dem Verfahren des primären Stössels, im Spritzenkörper bleibt. Der Spritzenkörper kann dazu zum Beispiel radial nach innen ragende, federnde Nasen umfassen. Weiter kann die Rückhaltevorrichtung als mindestens ein länglicher Abschnitt ausgebildet sein, welcher axial über ein proximales Ende federnd mit dem Spritzenkörper verbunden ist und in einem proximalen und einem distalen Bereich nach innen, in den Innenraum des Spritzenkörpers ragende Nasen aufweisen kann. Der Feststoff kann entweder zwischen den Nasen angeordnet oder durch eine oder mehrere Nasen festgeklemmt sein. Die Deaktivierung der Rückhaltevorrichtung erfolgt durch ein Zusammenwirken des primären Stössels mit der proximal angeordneten Nase, wodurch die distale Nase über den länglichen Abschnitt radial nach aussen geführt wird.

Alternativ kann der Spritzenkörper auch lediglich eine Querschnittsverengung aufweisen, so dass der Feststoff an dieser Stelle durch Reibschluss gehalten wird. Die Querschnittsverengung kann auch mittels in radialer Richtung federnde Elemente realisiert sein. Schliesslich könnte auf die Rückhaltevorrichtung auch verzichtet werden. In diesem Fall ist es von Vorteil, wenn die Vorrichtung einen Verschluss für die Kanüle umfasst, welche ein vorzeitiges Austreten des Feststoffes verhindern kann. Dies kann zum Beispiel durch eine einfach Kappe oder einen in die Kanüle eingeführten Stift realisiert werden. Dabei muss aber beachtet werden, dass nach dem Entfernen des Verschlusses die Kanüle nicht nach unten gerichtet werden sollte, da dadurch der Feststoff aus der Vorrichtung fallen könnte.

Vorzugsweise umfasst die Vorrichtung eine Zunge, insbesondere eine Zunge aus scharfkantigem Metall, welche mit dem Gehäuse verbunden ist und mit dem Spritzenkörper in Eingriff steht. Damit wird erreicht, dass der Spritzenkörper nicht in distale Richtung verfahren werden kann. Dies hat den Vorteil, dass damit auch die Kanüle während des Zurückziehens derselben nicht wieder zurück in den Körper verfahren werden kann. Weiter hat die Ausbildung einer Zunge den Vorteil, dass damit eine rasterlose distale Rückführverhinderung erreicht wird. Dies ist besonders vorteilhaft, da der Verwender der Vorrichtung den sekundären Stössel kontinuierlich einfahren kann. Die Ausbildung dieser Vorrichtung zum Blockieren einer Bewegungsrichtung kann ähnlich wie diejenige eines Kabelbinders ausgebildet sein.

Alternativ kann die Zunge auch mit dem sekundären Stössel zusammenwirken. Die Zunge kann auch entweder mit dem sekundären Stössel verbunden sein und mit dem Spritzenkörper zusammenwirken, oder umgekehrt. Weiter kann auch ein Element vorgesehen sein, welches mit einer der Zahnstangen zusammenwirkt und eine entsprechende Bewegung in eine Richtung blockieren kann. Schliesslich kann auch das Zahnrad so ausgebildet sein, dass es nur in eine Richtung drehbar ist, wobei das Zahnrad zum Beispiel als Klinkenrad, bekannt aus mechanischen Uhrwerken, ausgebildet sein kann. Vorzugsweise bestehen die Kanüle, die erste und die zweite Feder aus Stahl, wobei aber auch Kunststoffe eingesetzt werden können. Für die restlichen Teile der Vorrichtung können zum Beispiel Polymethylmethacrylat (PMMA), Polycarbonat oder weitere dem Fachmann bekannte Kunststoffe eingesetzt werden, wobei insbesondere auch für verschiedene Teile verschiedene Kunststoffe eingesetzt werden können.

Die Dimensionierung der Vorrichtung kann beliebig gewählt werden und insbesondere dem Feststoff angepasst sein. Die Eindringtiefe kann durch eine Wahl der Kanülenlänge reguliert werden.

Nachfolgend sei schrittweise ein möglicher Ablauf der Injektion eines Feststoffes mit einer Ausführungsform der erfindungsgemässen Vorrichtung beschreiben:
1. In einem ersten Schritt wird, falls vorhanden, eine Schutzkappe von der Kanüle entfernt und anschliessend die Kanüle in den Körper des zu Injizierenden eingeführt.
2. Nach dem Einführen der Kanüle wird das Betätigungselement im proximalen Bereich des sekundären Stössels betätigt, wodurch
   a. die zweite Verriegelungsvorrichtung entriegelt wird; und dadurch
   b. die erste Feder entspannt wird; wodurch
   c. der primäre Stössel durch die Federkraft der ersten Feder einen Feststoff aus dem Spritzenkörper in die Kanüle überführt; worauf
   d. der primäre Stössel zumindest teilweise in der Kanüle und der Feststoff vollständig ausserhalb des Gehäuses zu liegen kommt; und
   e. die erste Verriegelungsvorrichtung deaktiviert wird.
3. Anschliessend wird der sekundäre Stössel in distaler Richtung verfahren; wodurch
   a. der Spritzenkörper und/oder die Kanüle bei ortsfestem primären Stössel in proximaler Richtung verfahren wird, so dass die Kanüle, insbesondere vollständig, in den Spritzenkörper zurückgefahren wird; und
   b. die dritte Verriegelungsvorrichtung entriegelt wird; wodurch
   c. die zweite Feder entspannt und die Hülse teilweise in distaler Richtung aus dem Gehäuse verfahren wird und insbesondere den Körper kontaktiert.
4. Schliesslich wird die Spritze aus dem Körper entfernt, wobei insbesondere ausschliesslich der sekundäre Stössel aus dem Körper gezogen wird. Simultan wird dadurch die Hülse vollständig mittels der zweiten Feder über den aus dem Gehäuse ragenden Teil des Stössels geschoben, worauf die Hülse einrastet.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine schematische Darstellung eines Schnittes entlang der Längsachse einer Ausführungsform der erfindungsgemässen Vorrichtung;
- Fig. 2: eine Darstellung gemäss Figur 1 nach der Einführung der Kanüle in den Körper;
- Fig. 3: eine Darstellung gemäss Figur 2 nach der Betätigung des Betätigungselements;
- Fig. 4: eine Darstellung gemäss Figur 3 nach dem Einführen des sekundären Stössels; und
- Fig. 5: eine Darstellung gemäss Figur 4 nach vollzogener Injektion.

Grundsätzlich sind in den Figuren gleiche Teile mit gleichen Bezugszeichen versehen.

### Wege zur Ausführung der Erfindung

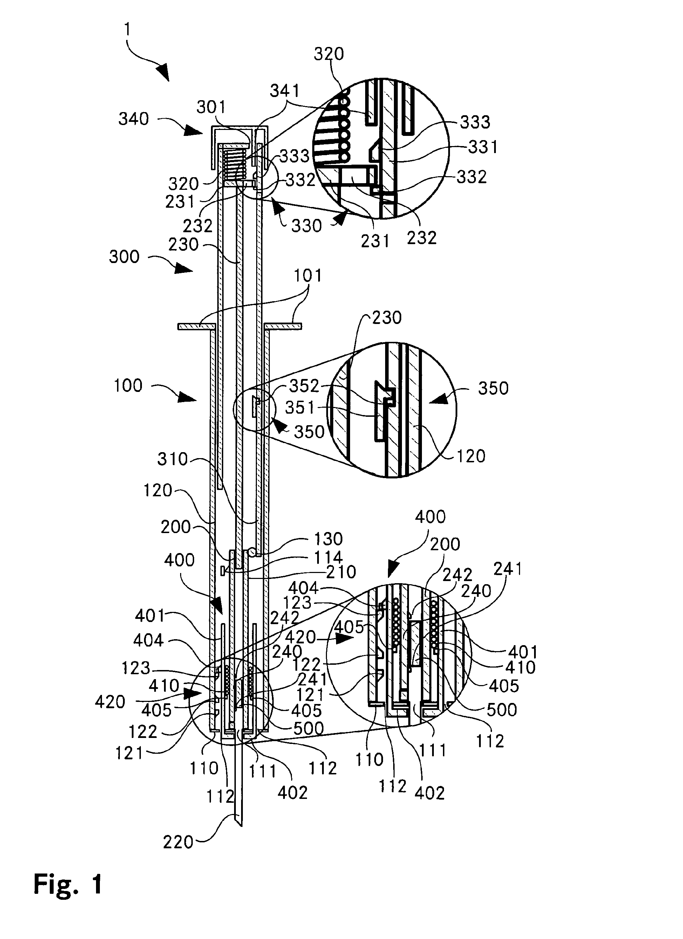

Die Figur 1 zeigt eine schematische Darstellung eines Schnittes entlang der Längsachse einer Ausführungsform der erfindungsgemässen Vorrichtung 1 vor der Verwendung.

Die Vorrichtung 1 umfasst ein Gehäuse 100, welches im Wesentlichen als einseitig geschlossener Hohlzylinder ausgebildet ist, wobei die Öffnung proximal orientiert ist. Die Vorrichtung 1 umfasst weiter einen primären Stössel 230 und einen Spritzenkörper 200, in welchem insbesondere vor der Verwendung der Vorrichtung 1 ein Feststoff 500 mittels einer Rückhaltevorrichtung gehalten ist. Der Spritzenkörper 200 ist fest mit einer Kanüle 220 verbunden, wobei der primäre Stössel 230 axial durch den Spritzenkörper 200 hindurch in die Kanüle 220 verfahrbar ist. Der Spritzenkörper 200 ist mit der Kanüle 220 im Gehäuse 100 axial verfahrbar gelagert. Weiter umfasst die Vorrichtung 1 einen sekundären Stössel 300, welcher ebenfalls axial im Gehäuse 100 verfahrbar gelagert ist und im Wesentlichen als einseitig geschlossener Hohlzylinder, mit einer distal orientierten Öffnung ausgebildet ist. Der primäre Stössel 230 ist axial verfahrbar im sekundären Stössel 300 gelagert. Schliesslich umfasst die Vorrichtung 1 eine Hülse 400, welche in einem distalen Bereich des Gehäuses 100 axial verfahrbar gelagert ist. Die Hülse 400 ist auch als ein im Wesentlichen einseitig geschlossener Hohlzylinder ausgebildet, wobei die Öffnung proximal orientiert ist.

Das Gehäuse 100 umfasst im Bereich der Öffnung zwei bezüglich einer Längsachse der Vorrichtung 1 rechtwinklig nach aussen ragende Halteelemente 101, welche insbesondere mittels Zeige- und Mittelfinger ergriffen werden können.

An einem Gehäuseboden 110 des Gehäuses 100 umfasst dieses eine zentrierte, kreisrunde Kanülenöffnung 111, durch welche eine Kanüle 220 durchführbar ist. Weiter sind am Gehäuseboden 110, die Kanülenöffnung 111 umgebend, mehrere kreisbogenförmige Schlitze als Hülsenöffnungen 112 ausgebildet, durch welche jeweils ein Hülsenmantelsegment eines Hülsenmantels 401 der Hülse 400 axial verfahrbar gehalten wird.

Ein Gehäusemantel 120 des Gehäuses 100 umfasst inwändig im Bereich des Gehäusebodens 110 drei zueinander beabstandete, axial hintereinander in einer Reihe orientierte, vorstehende Elemente 121 - 123 als Teile einer dritten Verriegelungsvorrichtung 420 (siehe unten). Diese vorstehenden Elemente 121 - 123 sind keilförmig ausgebildet, wobei eine abgeschrägte Flanke des dem Gehäuseboden 110 am nächsten liegenden vorstehenden Elements 121 distal orientiert ist. Das nachfolgende keilförmige vorstehende Element 122 ist in Bezug auf das erste Element 121 an einer senkrecht zur Gehäuselängsachse stehenden Ebene gespiegelt, das letzte keilförmige vorstehende Element 123, welches vom Gehäuseboden 110 am weitesten entfernt ist, ist wiederum wie das vorstehende Element 121 orientiert. Im Querschnitt senkrecht zur Gehäuselängsachse, sind die vorstehenden Elemente 121 - 123 nockenförmig ausgebildet.

Weiter umfasst das Gehäuse 100 ein Zahnrad 130, welches bezüglich einer axial bemessenen Länge des Gehäuses 100 ungefähr mittig und bezüglich einer Querschnittsfläche des Gehäuses 100 zum Mittelpunkt um ungefähr einen halben Spritzenkörperdurchmesser des Spritzenkörpers 200 parallel nach aussen versetzt rotierbar am Gehäuse 100 gelagert ist.

Distal zum und axial unter dem Zahnrad 130 ist mit dem Gehäuse 100 weiter eine Zunge 114 verbunden, welche bezüglich des Zahnrades 130 geringfügig radial nach innen versetzt ist und in distaler Richtung zum Spritzenkörper 200 ungefähr einen Winkel von 45° einschliesst. Die Zunge 114 ist vorzugsweise aus scharfkantigem Metall, insbesondere aus Stahl ausgebildet und wirkt mit einem zumindest geringfügig federnden Bereich mit dem Spritzenkörper 200 so zusammen, dass der Spritzenkörper 200 zwar proximal verfahren werden kann, aber ein Verfahren des Spritzenkörpers 200 in distaler Richtung verhindert wird. Bevorzugt wirkt die Zunge 114 mit einem glatten Bereich (nicht mit dem als Zahnstange ausgebildeten Bereich) des Spritzenkörpers 200 zusammen, so dass der sekundäre Stössel 300 kontinuierlich eingefahren werden kann.

Mit dem Gehäuse 100 ist weiter ein Verriegelungselement 351, als Teil einer ersten Verriegelungsvorrichtung 350 verbunden, welches entgegen einer Federkraft im Wesentlichen radial verschwenkbar ausgebildet ist. Das Verriegelungselement 351 ist als axial orientierter länglicher Abschnitt und in der vorliegenden Ausführungsform einstückig mit dem Gehäuse 100 ausgebildet. In einem distalen Bereich ist das Verriegelungselement 351 mit dem Gehäuse 100 fest verbunden und weist an einem proximalen Bereich ein radial abstehendes Element und am proximalen Ende eine abgeschrägte Flanke auf, welche in distaler Richtung einen Winkel von ungefähr 45° mit dem primären Stössel 230 einschliesst.

Der Spritzenkörper 200 umfasst eine axial orientierte erste Zahnstange 210 (nicht explizit dargestellt) an seiner Mantelfläche, welche mit dem Zahnrad 130 in einem ersten Zustand über ein proximales Ende in Eingriff steht. Weiter umfasst er eine aus der EP 09 405 186.9 (Forteq Nidau AG) bekannte Rückhaltevorrichtung zum Zurückhalten des Feststoffes 500. Dazu weist der Spritzenkörper 200 ein längliches Element 240 auf, welches proximal mit dem Spritzenkörper 200 federnd verbunden und als U-förmige Ausnehmung einstückig mit dem Spritzenkörper ausgebildet ist. Das längliche Element 240 weist radial in den Innenraum des Spritzenkörpers 200 ragende Nasen 241, 242 auf, wobei eine Nase 241 distal und die andere Nase 242 um mindestens eine Länge eines Feststoffes 500 proximal beabstandet angeordnet ist, so dass der Feststoff 500 zwischen den Nasen 241, 242 gehalten werden kann. Zur Deaktivierung der Rückhaltevorrichtung wird mittels des primären Stössels 230 die proximal angeordnete Nase 242 radial nach aussen getrieben, worauf simultan über den länglichen Abschnitt 240 die distal angeordnete Nase 241 radial nach aussen getrieben wird und so den Innenraum des Spritzenkörpers 200 für den Feststoff 500 frei gibt.

Der sekundäre Stössel 300 ist im Wesentlichen als einseitig geschlossener Zylinder ausgebildet, wobei die Zylinderöffnung distal angeordnet ist und der distale Bereich weiter einen zylindermantelsegmentförmigen Ausschnitt aufweist. Der sekundäre Stössel 300 weist an seinem proximalen im Wesentlichen geschlossenen Ende eine Stiftöffnung 301 und seitlich im Bereich des proximalen Endes eine zweite Verriegelungsvorrichtung 330 auf, welche ein federndes Element 331 umfasst. Das federnde Element 331 ist in der vorliegenden Ausführungsform als U-förmige Ausnehmung im sekundären Stössel 300 ausgebildet und proximal mit diesem verbunden und weist zwei nach innen ragende axial beabstandete Elemente 332, 333 auf, wobei das proximal angeordnete Element 333 keilförmig ausgebildet ist und eine abgeschrägte Flanke in proximaler Richtung aufweist. Im Querschnitt, senkrecht zur Gehäuselängsachse, sind die Elemente 332, 333 nockenförmig ausgebildet. Das federnde Element 331 ist radial nach aussen biegbar ausgebildet.

Der sekundäre Stössel 300 umfasst weiter ein im Wesentlichen als einseitig geschlossener Zylinder ausgebildetes Betätigungselement 340, welches parallel zum Zylindermantel einen mit dem Zylinderboden verbundenen Stift 341 aufweist. Das Betätigungselement 340 ist über das proximale Ende des sekundären Stössels 300 gestülpt, wobei der Stift 341 durch die Stiftöffnung 301 geführt ist und in einem ersten Zustand axial proximal bezüglich des Elements 333 angeordnet ist, insbesondere ist das Betätigungselement 340 im ersten Zustand nicht vollständig eingefahren. Während die Halteelemente 101 mit Zeige- und Mittelfinger ergriffen werden, erfolgt die Betätigung des Betätigungselements 340 typischerweise mit dem Daumen, nämlich indem der Daumen in Richtung des Zeige-und Mittelfingers bewegt und damit der sekundäre Stössel 300 distal verfahren wird.

Der sekundäre Stössel 300 ist vom distalen Bereich aus an der Innenwand mit einer axial orientierten zweiten Zahnstange 310 ausgestattet, welche insbesondere einstückig mit dem sekundären Stössel 300 ausgebildet ist und welche mit dem Zahnrad 130 des Gehäuses zusammenwirken kann. In einem ersten Zustand steht ein distaler Bereich der zweiten Zahnstange 310 mit dem Zahnrad 130 in Eingriff. Damit ist die Bewegung des sekundären Stössels 300 mit derjenigen des Spritzenkörpers 200 gekoppelt, wobei eine distale Bewegung des sekundären Stössels 300 über das so gebildete Zahnstangengetriebe in eine proximale Bewegung des Spritzenkörpers 200 umgewandelt wird und wobei eine umgekehrte Bewegung durch die Zunge 114 verhindert wird.

Der im sekundären Stössel 300 geführte primäre Stössel 230 weist an seinem proximalen Ende einen scheibenförmigen Stösselkopf 231 mit einer Öffnung 232 auf. Der Durchmesser des Stösselkopfes 231 entspricht im Wesentlichen dem Innendurchmesser des sekundären Stössels 300. Der Stösselkopf 231 und der sekundäre Stössel 300 schliessen eine erste Feder 320 ein, welche in einem ersten Zustand gespannt ist. Diese erste Feder 320 ist als Schraubenfeder ausgebildet und besteht aus Stahl. Der Stösselkopf 231 ist zwischen den Elementen 332, 333 des sekundären Stössels 300 gehalten, insbesondere wirkt durch die gespannte erste Feder 320 eine Kraft in distaler Richtung auf das Element 332.

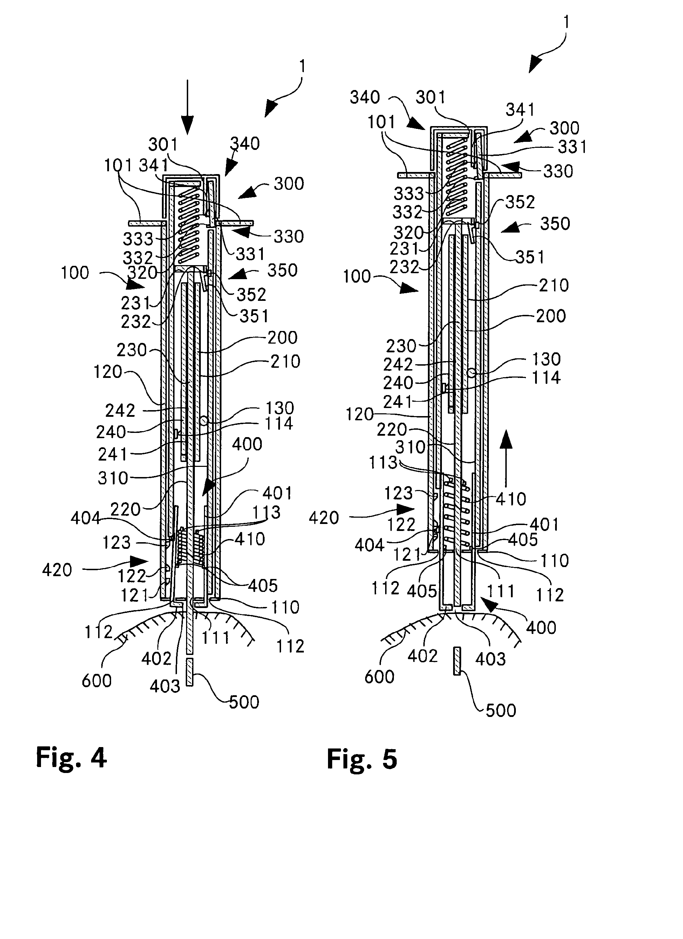

Die im Gehäuse axial verfahrbar gelagerte Hülse 400, welche als im Wesentlichen einseitig geschlossener Hohlzylinder ausgebildet ist, umfasst an einem Hülsenboden 402 der Hülse 400 eine Kanülenöffnung 403 für die Kanüle 220. Der Hülsenmantel 401 weist Einschnitte auf (nicht ersichtlich), welche der Hülsenöffnung 112 des Gehäuses 100 entsprechend ausgebildet sind und ist in der Hülsenöffnung 112 des Gehäuses axial verfahrbar geführt. Das Gehäuse 100 weist radial nach innen ragende Rückhalteelemente 113 (ersichtlich in Figuren 4 und 5) auf, welche durch die Einschnitte des Hülsenmantels 401 in den Innenraum der Hülse 400 ragen. Die Innenseite des Hülsenbodens 402 kontaktiert in einem ersten Zustand die Aussenseite des Gehäusebodens 101. Eine zweite Feder 410 ist über einen proximalen Bereich durch die Rückhaltelemente 113 des Gehäuses 100 und über einen distalen Bereich durch Rückhalteelemente 405 in der Hülse 400 gehalten und im ersten Zustand gespannt. Die zweite Feder 410 ist als Schraubenfeder ausgebildet und besteht aus Stahl. Der Hülsenmantel 401 ist aussenseitig mit einem keilförmigen vorstehenden Element 404 mit einem quaderförmigen Aufsatz verbunden, wobei die abgeschrägte Flanke des vorstehenden Elements 404 proximal orientiert ist.

In den nachfolgenden Abschnitten wird nun die Verwendung der Vorrichtung 1 zur Injektion eines Feststoffes anhand der **Figuren 1 bis 5** näher erläutert.

Die Vorrichtung 1 ist vor der Verwendung im Zustand gemäss **Figur 1**.

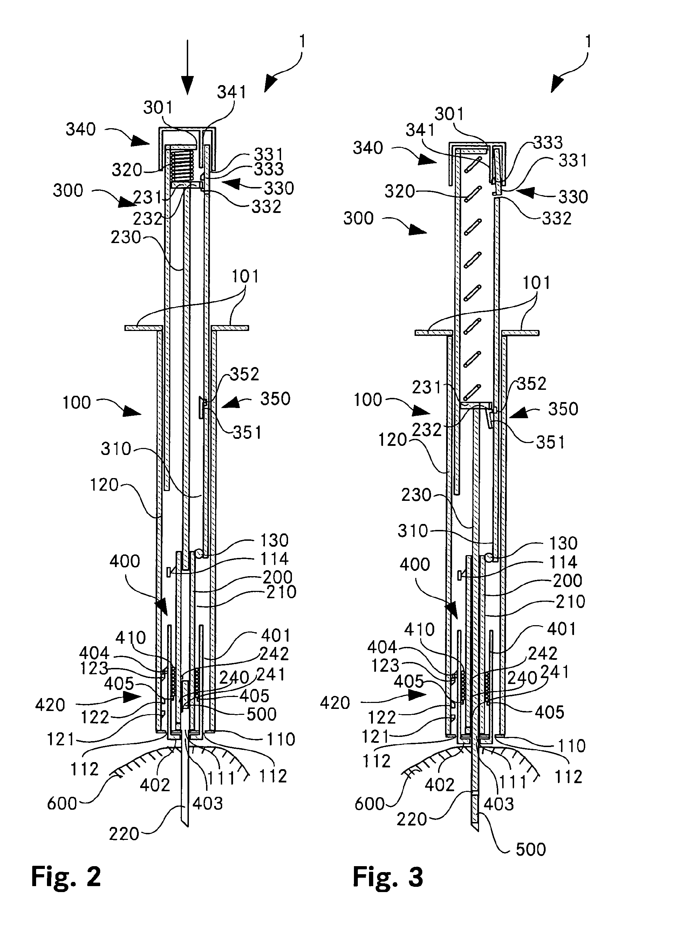

Die **Figur 2** zeigt eine schematische Darstellung einer Ausführungsform der erfindungsgemässen Vorrichtung 1 gemäss Figur 1 nach der Einführung der Kanüle 220 in einen Körper 600, insbesondere als erster Schritt bei der Injektion eines Feststoffes, vor der Betätigung des Betätigungselements 340.

Die Vorrichtung 1 wird nun durch den Verwender mit dem Zeige- und dem Mittelfinger an den Halteelementen 101 ergriffen und der Daumen wird vorerst drucklos auf das Betätigungselement 340 gelegt.

Die **Figur 3** zeigt eine schematische Darstellung gemäss Figur 2 nach der Betätigung des Betätigungselements 340. Wird nun das Betätigungselement 340 mittels des Daumens in distaler Richtung betätigt, so wird damit der Stift 341 distal verfahren und kontaktiert alsdann die Flanke des Elementes 333 und biegt so das Verriegelungselement 331 radial nach aussen. Damit gekoppelt wird auch das Element 332 radial nach aussen getrieben, wodurch das Element 332 nicht mehr in Eingriff mit dem Stösselkopf 231 des primären Stössels 230 steht. Damit wird der Weg für den primären Stössel 230 in distale Richtung freigegeben. Anschliessend erfolgt eine Entspannung der ersten Feder 320, wodurch der primäre Stössel 230 in distaler Richtung relativ zum sekundären Stössel 300 verfahren wird.

Das distale Ende des primären Stössels 230 erreicht im Spritzenkörper 200 zuerst die erste Nase 242 und treibt diese radial nach aussen. Damit gekoppelt werden der längliche Abschnitt 240 und damit auch die Nase 241 radial nach aussen getrieben, wodurch der Weg für den Feststoff 500 freigegeben wird. Der primäre Stössel 230 erfasst danach, angetrieben durch die noch teilweise gespannte erste Feder 320, mit seinem distalen Ende den Feststoff 500 und führt diesen in die Kanüle 220, insbesondere in einen Bereich der Kanüle 220 welcher ausserhalb des Gehäuses 100 liegt. Der Abstand des Feststoffes zum Gehäuse 100, respektive zur Kanülenöffnung 403 der Hülse 400, entspricht dabei im Wesentlichen einer vorgesehenen Einpflanztiefe des Feststoffes 500 im Körper 600.

In diesem Zustand befindet sich also die Kanüle 220 mit einem wesentlichen Bereich im Körper 600, ein distaler Bereich des primären Stössels 230 in der Kanüle 220 und damit auch im Körper 600 und der Feststoff 500 befindet sich vor dem distalen Ende des primären Stössels 230 innerhalb des Körpers 600.

Gleichzeitig mit dem vollständigen Einfahren des primären Stössels 230 kontaktiert der Stösselkopf 231 das Verriegelungselement 351 der ersten Verriegelungsvorrichtung 350. Insbesondere wird durch die Öffnung 232 des Stösselkopfes 231 die abgeschrägte Flanke des Verriegelungselements 351 radial nach innen getrieben, womit ein radial nach aussen ragendes und vorgängig in einer Ausnehmung 352 des sekundären Stössels liegendes vorstehendes Element des Verriegelungselements 351 radial nach innen aus dieser Ausnehmung 352 gehoben wird. Damit wird die erste Verriegelungsvorrichtung 350 entriegelt, so dass der sekundäre Stössel 300 im Gehäuse 100 axial verfahren werden kann. Das Einführen des sekundären Stössels 300 erfolgt wiederum mittels eines durch den Daumen ausgeübten Druckes auf das Betätigungselement 340.

Wird nun der sekundäre Stössel 300 mittels eines Druckes auf das Betätigungselement 340 in das Gehäuse 100 in distale Richtung eingefahren, so erfolgt gleichzeitig über die erste und die zweite Zahnstange 210, 310, welche über das Zahnrad 130 gekoppelt sind, eine proximale Bewegung des Spritzenkörpers 200 und damit auch der Kanüle 220, währenddessen der primäre Stössel 230 ortsfest bleibt. Damit wird die Kanüle 220 über den Feststoff 500 und den aus dem Gehäuse 100 ragenden Bereich des primären Stössels 230 hinweggeführt, so dass der Feststoff 500 ausserhalb verbleibt und schliesslich nur noch ein Bereich des primären Stössels 230 aus dem Gehäuse hinausragt.

Die **Figur 4** zeigt eine schematische Darstellung gemäss Figur 3 nach dem Einführen des sekundären Stössels 300. Beim Einfahren des sekundären Stössels 300 mittels Betätigung des Betätigungselements 340 wird die erste Feder 320 wieder komprimiert. Der Feststoff 500 und ein distaler Bereich des primären Stössels 230 verbleiben im Körper 600, während die Kanüle 220 über den Feststoff 500 und über den distalen Bereich des primären Stössels 230 zurückgezogen wird. Der Feststoff 500 wird derweilen durch den ortsfesten primären Stössel 230 im Körper 600 zurückgehalten.

Damit bei einem Loslassen des sekundären Stössels 300 dieser mittels der ersten Feder 320 nicht proximal verfahren kann, ist die Zunge 114 vorgesehen, welche federnd auf die Mantelfläche des Spritzenkörpers 200 einwirkt und so bei einer proximalen auf den sekundären Stössel 300 wirkenden Kraft mit dem Spritzenkörper 200 verkantet und damit ein Zurückfahren des sekundären Stössels 300, respektive ein distales Verfahren der Kanüle 220 wieder in den Körper 600 verhindert.

Beim vollständigen Einfahren des sekundären Stössels 300 kontaktiert dieser mit dem Bereich des zylindermantelsegmentförmigen Ausschnittes das vorstehende Element 404 der Hülse 400, womit das vorstehende Element 404 radial nach innen getrieben und dadurch über das vorstehende Element 123 des Gehäuses 100 gehoben wird. Dadurch wird die dritte Verriegelungsvorrichtung 420 entriegelt, worauf sich die zweite Feder 410 entspannen und so die Hülse 400 teilweise aus dem Gehäuse 100, insbesondere über den aus dem Gehäuse 100 hinausragenden Bereich des primären Stössels 230 hinausfahren kann. Allerdings befindet sich in diesem Zustand der über das Gehäuse 100 herausragende Bereich des primären Stössels 230 noch immer im Körper 600, wodurch die Hülse 400 in einem ersten Moment mittels der zweiten Feder 410 an den Körper 600 gepresst wird und damit nicht vollständig herausfahren kann.

Die **Figur 5** zeigt eine schematische Darstellung gemäss Figur 4 nach vollzogener Injektion. Nachdem die dritte Verriegelungsvorrichtung 420 entriegelt ist, wird die Vorrichtung vorsichtig aus dem Körper 600 gezogen, insbesondere wird der distale Bereich des primären Stössels 230 aus dem Körper 600 gezogen, während der Feststoff 500 in demselben verbleibt. Gleichzeitig kann sich nun die zweite Feder 410 vollständig entspannen und so die Hülse 400 aus dem Gehäuse 100 hinaus über den primären Stössel 230 geführt werden. Das vorstehende Element 404 der Hülse 400 erreicht währenddessen das nächste vorstehende Element 122 des Gehäuses 100 und hebt sich aufgrund der keilförmigen Ausbildung des vorstehenden Elements 122 mittels der Federkraft über dasselbe hinweg und rastet zwischen den vorstehenden Elementen 122 und 121, insbesondere irreversibel ein. In diesem Zustand ist der aus dem Gehäuse 100 ragende Bereich des primären Stössels 230 vollständig durch die Hülse 400 abgedeckt, womit mögliche Kontaminationen unterbunden werden können.

Die Kanüle 220, die erste Feder 320 sowie die zweite Feder 410 bestehen aus Metall, insbesondere aus Stahl. Die übrigen Teile der Vorrichtung 1 sind aus einem Kunststoff, zum Beispiel aus Polymethylmethacrylat (PMMA) oder Polycarbonat ausgebildet. Dem Fachmann sind auch weitere geeignete Materialien bekannt, welche für einzelne Teile der Vorrichtung 1 eingesetzt werden können. Zum Beispiel könnten die erste und die zweite Feder 320, 410 auch aus einem Kunststoff ausgebildet sein.

Die Halteelemente 101 können auch als eine um das Gehäuse umlaufend ausgebildete Kreisscheibe oder als weitere dem Fachmann bekannte Halteelemente 101 ausgebildet sein. Insbesondere kann auch eine ergonomisch angepasste Form der Halteelemente 101 vorgesehen sein.

Auch die vorstehenden Elemente 121 - 123, und/oder die Zahnstangen 210, 310 können umlaufend ausgebildet sein. Durch eine umlaufende Ausbildung der vorstehenden Elemente 121 - 123, respektive der Zahnstangen 210, 310 kann die Vorrichtung bezüglich Rotationen des Gehäuses, des sekundären Stössels, der Hülse und des Spritzenkörpers relativ zueinander resistent ausgebildet werden.

Die Zunge 114 kann auch mit dem Zahnrad 130 oder mit dem sekundären Stössel 300 zusammenwirken.

Zusammenfassend ist festzustellen, dass durch die Erfindung eine Vorrichtung zum Injizieren von Feststoffen geschaffen wird, welche besonders einfach in der Handhabung ist und auch durch ungeübte Benutzer mühelos und intuitiv verwendet werden kann.

## Patentansprüche

1. Vorrichtung (1) zum Injizieren eines Feststoffes (500) in einen menschlichen oder einen tierischen Körper (600), insbesondere eine Spritze für die Injizierung eines Feststoffmedikaments, umfassend:
a) ein Gehäuse (100);
b) einen Spritzenkörper (200) zur Aufnahme des Feststoffes (500), verbunden mit einer Kanüle (220);
c) einen primären Stössel (230), welcher im Spritzenkörper (200) und in der Kanüle (220) verfahrbar ist;
d) einen sekundären Stössel (300), welcher im Gehäuse (100) verfahrbar ist;
**dadurch gekennzeichnet, dass**
e) die Vorrichtung (1) einen Kraftübertragungsmechanismus umfasst, welcher zwischen dem sekundären Stössel (300) und dem Spritzenkörper (200) und/oder der Kanüle (220) wirken kann; wobei
f) durch eine Betätigung des sekundären Stössels (300) in distaler Richtung ein Verfahren des Spritzenkörpers (200) und/oder der Kanüle (220) in proximaler Richtung bewirkbar ist.

2. Vorrichtung (1) nach Anspruch 1, **dadurch gekennzeichnet, dass** der Kraftübertragungsmechanismus ein Zahnstangengetriebe umfasst.

3. Vorrichtung (1) nach Anspruch 1 oder 2, **dadurch gekennzeichnet, dass** der Kraftübertragungsmechanismus als Zahnstangengetriebe ausgebildet ist, welches eine erste Zahnstange (210), eine zweite Zahnstange (310) und ein Zahnrad (130) umfasst, wobei
a) das Zahnrad (130) frei rotierbar im Gehäuse (100) gelagert ist; und
b) die erste Zahnstange (210) mit dem Spritzenkörper (200) verbunden ist; und
c) die zweite Zahnstange (310) mit dem sekundären Stössel (300) verbunden ist; wobei
d) das Zahnrad (130) mit der ersten und der zweiten Zahnstange (210, 310) zusammenwirkt.

4. Vorrichtung (1) nach einem der Ansprüche 1 bis 3, **dadurch gekennzeichnet, dass** sie eine erste Verriegelungsvorrichtung (350) umfasst, womit der sekundäre Stössel (300) bezüglich einer axialen Verfahrbarkeit im Gehäuse (100) blockierbar ist.

5. Vorrichtung (1) nach Anspruch 4, **dadurch gekennzeichnet, dass** die erste Verriegelungsvorrichtung (350) durch den primären Stössel (230), insbesondere durch eine distale Verschiebung des primären Stössels (230), deaktivierbar ist.

6. Vorrichtung (1) nach einem der Ansprüche 1 bis 4, **dadurch gekennzeichnet, dass** sie einen ersten Energiespeicher umfasst, wobei Energie des ersten Energiespeichers zur distalen Verschiebung des primären Stössels (230) einsetzbar ist.

7. Vorrichtung (1) nach Anspruch 6, **dadurch gekennzeichnet, dass** der erste Energiespeicher eine erste Feder (320), insbesondere zur Verschiebung des primären Stössels (230) in distaler Richtung umfasst, wobei die erste Feder (320) insbesondere als Schraubenfeder ausgebildet ist.

8. Vorrichtung (1) nach einem der Ansprüche 1 bis 7, **dadurch gekennzeichnet, dass** der primäre Stössel (230) im sekundären Stössel (300) geführt ist.

9. Vorrichtung (1) nach Anspruch 7, **dadurch gekennzeichnet, dass** die erste Feder (320) im sekundären Stössel (300) so angeordnet ist, dass der primäre Stössel (230) durch eine Federkraft der ersten Feder (320) aus dem sekundären Stössel (300) axial verfahrbar ist.

10. Vorrichtung (1) nach einem der Ansprüche 7 bis 9, **dadurch gekennzeichnet, dass** sie eine zweite Verriegelungsvorrichtung (330) umfasst, mit welcher der primäre Stössel (230) bezüglich des sekundären Stössels (300), insbesondere bei gespannter erster Feder (320), verriegelbar ist.

11. Vorrichtung (1) nach Anspruch 10, **dadurch gekennzeichnet, dass** der sekundäre Stössel (300) ein Betätigungselement (340) umfasst, mit welchem die zweite Verriegelungsvorrichtung (330) von Hand entriegelbar ist, wobei insbesondere das Betätigungselement (340) an einem proximalen Ende des sekundären Stössels (300) angeordnet und bevorzugt als Druckknopf ausgebildet ist.

12. Vorrichtung (1) nach einem der Ansprüche 1 bis 11, **dadurch gekennzeichnet, dass** sie eine Hülse (400) umfasst, welche axial verfahrbar im distalen Ende des Gehäuses (100) gelagert ist und in einem ersten Zustand im Wesentlichen innerhalb des Gehäuses (100) angeordnet ist und in einem zweiten Zustand zumindest teilweise aus dem Gehäuse (100) hinausragt.

13. Vorrichtung (1) nach Anspruch 13, **dadurch gekennzeichnet, dass** ein zweiter Energiespeicher eine zweite Feder (410), insbesondere eine Schraubenfeder (410) umfasst, welche zwischen dem Gehäuse (100) und der Hülse (400) wirkbar ist und insbesondere im ersten Zustand gespannt ist.

14. Vorrichtung (1) nach Anspruch 12 oder 13, **dadurch gekennzeichnet, dass** sie eine dritte Verriegelungsvorrichtung (420) umfasst, womit die Hülse (400) bezüglich einer axialen Verfahrbarkeit im Gehäuse (100) verriegelbar ist.

15. Vorrichtung (1) nach Anspruch 14, **dadurch gekennzeichnet, dass** die dritte Verriegelungsvorrichtung (420) im ersten Zustand entriegelbar und insbesondere im zweiten Zustand nicht entriegelbar ist.

16. Vorrichtung (1) nach Anspruch 14 oder 15, **dadurch gekennzeichnet, dass** die dritte Verriegelungsvorrichtung (420) durch den sekundären Stössel (300) deaktivierbar ist.

17. Vorrichtung (1) nach einem der Ansprüche 1 bis 16, **dadurch gekennzeichnet, dass** der Spritzenkörper (200) eine Rückhaltevorrichtung zum Zurückhalten des Feststoffes (500) vor der Injektion umfasst.

18. Vorrichtung (1) nach einem der Ansprüche 1 bis 17, **dadurch gekennzeichnet, dass** in einem Zustand, insbesondere nach der Verwendung der Vorrichtung (1), die Kanüle (220) vollständig innerhalb des Gehäuses (100) liegt; der primäre Stössel (230) distal und die Hülse (400) teilweise aus dem Gehäuse (100) hinausragen, wobei die Hülse (400) einen aus dem Gehäuse (100) hinausragenden Bereich des primären Stössels (230) vollständig abdeckt.
